# EUROPEAN PATENT SPECIFICATION

(11) **EP 4 116 709 B1**
(45) Date of publication and mention of the grant of the patent: **18.06.2025**
(21) Application number: 21814369.1
(22) Date of filing: 14.05.2021
(51) Int. Cl.: G01N 33/483, G01N 33/53, G01N 33/543, G01N 33/566, G01N 27/02

(54) **INSULIN DETECTION METHOD AND DETECTION KIT**
INSULINNACHWEISVERFAHREN UND NACHWEISKIT
MÉTHODE DE DÉTECTION D'INSULINE ET KIT DE DÉTECTION

(30) Priority: 29.05.2020 JP 2020094490
(43) Date of publication of application: 11.01.2023
(73) Proprietor: Hiroshima University, Higashihiroshima-shi Hiroshima 739-8511 (JP); TERUMO Kabushiki Kaisha, Tokyo 151-0072 (JP)
(72) Inventor: FUNABASHI, Hisakage, Higashihiroshima-shi, Hiroshima 739-8511 (JP); KURODA, Akio, Higashihiroshima-shi, Hiroshima 739-8511 (JP); SATO, Hiroya, Nakakoma-gun, Yamanashi 409-3853 (JP)
(74) Representative: Casalonga
(86) International application number: PCT/JP2021/018405
(87) International publication number: WO 2021/241277

(56) References cited:
- JP-A- 2016 109 666
- JP-A- 2018 504 597
- US-A1- 2015 177 180
- SHIGETO HAJIME ET AL: "A BRET-Based Homogeneous Insulin Assay Using Interacting Domains in the Primary Binding Site of the Insulin Receptor", ANALYTICAL CHEMISTRY, vol. 87, no. 5, 18 February 2015 (2015-02-18), US, pages 2764 - 2770, XP093075343, ISSN: 0003-2700, DOI: 10.1021/ac504063x
- LUONG ALBERT-DONALD ET AL: "Analytical and biosensing platforms for insulin: A review", SENSORS AND ACTUATORS REPORTS, vol. 3, 1 November 2021 (2021-11-01), pages 100028, XP093075339, ISSN: 2666-0539, Retrieved from the Internet <URL:https://www.sciencedirect.com/science/article/pii/S2666053921000047/pdfft?md5=d935cedf3b7809a088406c2d596a29db&pid=1-s2.0-S2666053921000047-main.pdf> DOI: 10.1016/j.snr.2021.100028
- MURAI YUSUKE ET AL: "Electrochemical Detection of Insulin with a Novel Protein-Based Artificial Receptor", ECS MEETING ABSTRACTS, 1 November 2020 (2020-11-01), XP093075359, Retrieved from the Internet <URL:https://iopscience.iop.org/article/10.1149/MA2020-02442821mtgabs> [retrieved on 20230822]
- MENTING, JOHN G. ET AL.: "How insulin engages its primary binding site on the insulin receptor", NATURE, vol. 493, pages 241 - 245, XP055262772, DOI: 10.1038/nature11781

## Description

### Technical Field

The present disclosure relates to an insulin detection method and an insulin detection kit.

### Background Art

Diabetes is a disease in which a high glucose concentration in blood (blood glucose level) continues due to a decrease in an action or insufficient secretion of insulin. When a hyperglycemia state continues for a long period of time, the hyperglycemia state not only causes capillaropathy such as nephropathy, retinopathy, and neuropathy, but also becomes a risk factor of macroangiopathy such as myocardial infarction and cerebral infarction. Therefore, in order to optimize diagnosis, prevention, and treatment of diabetes, it is useful to grasp not only the blood glucose level but also a blood component concentration of a factor related to diabetes. For example, JP-A-2016-109666 reports an insulin detection method including detecting fluorescence generated by luminescence resonance energy transfer generated between a luminescent and a fluorescent by using a first complex including an α-CT segment of an insulin receptor and the luminescent or the fluorescent and a second complex including an L1 domain of the insulin receptor and the fluorescent or the luminescent.

### Summary of Invention

According to the method disclosed in JP-A-2016-109666 above, insulin can be accurately quantified, but an insulin detection limit concentration was 50 nM or 800 nM (paragraph [0148]). On the other hand, an insulin concentration in a biological sample is considerably lower than that described above, and a method capable of detecting insulin at a lower concentration is sought. Shigeto et al. (Anal Chem; 2015 Mar 3; 87(5) : 2764-70) relates to detecting insulin using a BRET-based homogeneous insulin assay.

Accordingly, the present disclosure is made in view of the above circumstances, and an object of the present disclosure is to provide a method capable of detecting a trace amount of insulin contained in a sample.

The present inventors conduct intensive studies in order to solve the above problems. As a result, the present inventors found that insulin can be electrochemically detected by using an electrode on which an insulin binding protein including an α-CT segment and an L1 domain of an insulin receptor is immobilized, and the present invention was completed.

That is, the above object can be achieved by an insulin detection method including: adding a sample to an electrode having an insulin binding protein immobilized on a surface of the electrode, the insulin binding protein specifically recognizing insulin; and detecting an electrochemical change associated with formation of a complex of the insulin and the insulin binding protein. The insulin binding protein includes a first region that includes an α-CT segment of an insulin receptor and does not include a β subunit of the insulin receptor and a second region that includes an L1 domain of the insulin receptor and does not include the β subunit of the insulin receptor, and one of the first region and the second region is immobilized on the surface of the electrode.

### Brief Description of Drawings

[Fig. 1] Fig. 1 is a schematic view showing an electrode according to Example 1.
[Fig. 2] Fig. 2 is a schematic view showing an electrode according to Example 2.
[Fig. 3] Fig. 3 shows a measurement result of an insulin concentration using the electrode according to Example 1.
[Fig. 4] Fig. 4 shows a measurement result of an insulin concentration using the electrode according to Example 2.

### Description of Embodiments

Embodiments of the present disclosure will be described below. This present disclosure is not limited to the following embodiments.

According to a first aspect of the present disclosure, there is provided an insulin detection method including: adding a sample to an electrode having an insulin binding protein immobilized on a surface of the electrode, the insulin binding protein specifically recognizing insulin; and detecting an electrochemical change associated with formation of a complex of the insulin and the insulin binding protein. The insulin binding protein includes a first region that includes an α-CT segment of an insulin receptor and does not include a β subunit of the insulin receptor and a second region that includes an L1 domain of the insulin receptor and does not include the β subunit of the insulin receptor, and one of the first region and the second region is immobilized on the surface of the electrode.

According to the method of the present disclosure, a trace amount of insulin contained in a sample can be detected.

According to a second aspect of the present disclosure, there is provided an insulin detection kit including an electrode having an insulin binding protein immobilized on a surface of the electrode, the insulin binding protein specifically recognizing a site of insulin. The insulin binding protein includes a first region that includes an α-CT segment of an insulin receptor and does not include a β subunit of the insulin receptor and a second region that includes an L1 domain of the insulin receptor and does not include the β subunit of the insulin receptor, and one of the first region and the second region is immobilized on the surface of the electrode.

According to the detection kit of the present disclosure, a trace amount of insulin contained in a sample can be detected.

In the present specification, the "α-CT segment of an insulin receptor" is also simply referred to as the "α-CT segment", the "α-CT", or the "αCT". The "β subunit of the insulin receptor" is also simply referred to as the "β subunit". Further, the "first region that includes an α-CT segment of an insulin receptor and does not include a β subunit of the insulin receptor" is also simply referred to as the "first region". Similarly, in the present specification, the "L1 domain of the insulin receptor" is also simply referred to as the "L1 domain" or the "L1". The "second region that includes an L1 domain of the insulin receptor and does not include the β subunit of the insulin receptor" is also simply referred to as the "second region". Further, the electrode before the insulin binding protein that specifically recognizes the site of insulin is immobilized on the surface of the electrode is also simply referred to as the "electrode". The electrode having the insulin binding protein that specifically recognizes the site of insulin immobilized on the surface of the electrode is also simply referred to as the "insulin binding protein-immobilized electrode".

In the present specification, "X to Y" indicating a range includes X and Y, and means "X or more and Y or less". "nM" means nmol/L. Unless otherwise specified, an operation and measurement of physical properties and the like are performed at room temperature (20°C to 25°C).

### <Insulin Detection Method (First Aspect of Present Disclosure)>

According to the first aspect of the present disclosure, there is provided an insulin detection method including: adding a sample to an electrode having an insulin binding protein immobilized on a surface of the electrode, the insulin binding protein specifically recognizing insulin; and detecting an electrochemical change associated with formation of a complex of the insulin and the insulin binding protein. The insulin binding protein includes a first region and a second region, and one of the first region and the second region is immobilized on the surface of the electrode.

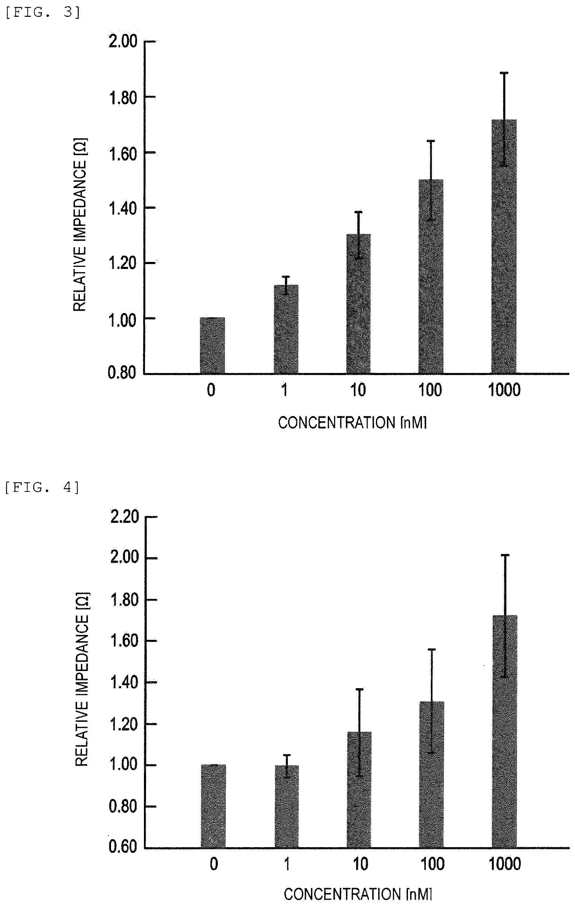

According to the method of the present disclosure, the insulin binding protein immobilized on the surface of the electrode specifically binds to insulin to form a complex of the insulin and the insulin binding protein (hereinafter also referred to as the "insulin-insulin binding protein complex"). That is, in the method of the present disclosure, by immobilizing the insulin binding protein including the first region and the second region in a primary structure on the surface of the electrode, it is possible to efficiently capture a change on the surface of the electrode associated with formation of the complex of the insulin and the insulin binding protein. In a recognition mechanism of the insulin by the insulin binding protein, first, the α-CT segment recognizes the insulin and binds to the insulin. Next, the L1 domain recognizes the insulin and the α-CT segment bound to the insulin to form the insulin-insulin binding protein complex. Therefore, in the method of the present disclosure, the insulin and the insulin binding protein bind to each other at least between insulin-α-CT segment and between insulin-α-CT segment-L1 domain to form the complex. In a process of forming the complex, a structural change occurs in the insulin binding protein. When a voltage is applied to the insulin binding protein-immobilized electrode, it is possible to detect a change in a state of the surface of the electrode which is induced by the structural change of the insulin binding protein associated with recognition and binding of the insulin. More specifically, when the complex is formed, electricity is less likely to flow (for example, a resistance value increases) as compared with the case in which the insulin binding protein is not bound to the insulin. By measuring this state change as an electrical change (for example, a change in impedance) by an electrochemical method (for example, electrochemical impedance spectroscopy using [Fe (CN)₆]⁻⁴/[Fe (CN) ₆] ⁻³), the insulin can be detected even in a trace amount (for example, less than 50 nM) (see Figs. 3 and 4). An insulin concentration can be calculated by performing appropriate correction as necessary from the above electrical change. Therefore, according to the method of the present disclosure, the insulin concentration in the sample can be quantified. Detection by the electrochemical method can be performed in a fairly short time (for example, about 1 minute to 30 minutes), and in addition, a detection device can be designed to be more compact than in a case of using an optical method. Therefore, the method of the present disclosure is suitable for point of care testing (POCT) performed at a site of medical care, such as a clinic, home, an examination room or a bed side, an operating room, or an ICU in a hospital.

The detection method according to the present disclosure includes both examining presence or absence of the insulin (test method) and measuring an amount of insulin contained in the sample (measurement method).

### (Sample Containing Insulin)

The sample used in the method of the present disclosure is used as a detection target for examining the presence or absence of the insulin or for measuring the amount of insulin contained in the sample. Examples of the sample include, but are not limited to, test samples (biological samples such as blood, plasma, serum, tissue, interstitial fluid, urine, and lymph), cells (cultured cells, cell lines, and the like), culture supernatant thereof, extracts thereof, partial purified fractions thereof, and artificially prepared samples (for example, drugs such as diabetes therapeutic agents and insulin aqueous solutions). An origin of the test sample is also not particularly limited, and may be derived from human or mammals other than human (for example, rodents such as rats, mice, hamsters, and guinea pigs; domestic animals such as sheep, pigs, cows, and horses; pets such as rabbits, cats, and dogs; primates such as rhesus monkey, green monkeys, cynomolgus monkeys, and chimpanzees). Among these, in consideration of benefits (for example, for diagonostic purpose), the sample containing insulin is preferably a test sample, more preferably blood (for example, whole blood, plasma, and serum), interstitial fluid and urine. That is, according to a preferred aspect of the present disclosure, the sample is selected from a group consisting of blood, interstitial fluid, and urine.

The sample may be used as it is without being purified (extracted), or may be used after being purified (extracted). For example, a test sample such as biological components such as blood, plasma, serum, tissue, interstitial fluid, urine, and lymph of a subject may be used. At this time, the test sample may be diluted, purified, or the like. Alternatively, a known method of purifying a protein or a method obtained by appropriately modifying the method of purifying a protein can be used. Specifically, for example, by homogenizing mammalian tissues or cells in the presence of an appropriate buffer and subjecting an obtained crude extract fraction of the tissue to chromatography such as reverse phase chromatography, ion exchange chromatography, and affinity chromatography, a purified product may be used as the sample. The insulin contained in the sample may be endogenous or exogenous insulin. That is, the sample may contain insulin derived from a commercially available insulin preparation.

### (First Region Including α-CT Segment of Insulin Receptor and not Including β Subunit of Insulin Receptor (First Region))

The first region including the α-CT segment of the insulin receptor and not including the β subunit of the insulin receptor (first region) is a polypeptide that includes the α-CT segment of the insulin receptor. The α-CT segment is a partial region of an α subunit, which is an extracellular domain of the insulin receptor, has insulin binding ability, and does not include a transmembrane region. A specific configuration of the α-CT segment in the present disclosure is not particularly limited, and any known α-CT segment may be adopted.

The first region according to the present disclosure does not include the β subunit of the insulin receptor. The β subunit is an intracellular domain of the insulin receptor, and includes a transmembrane region and a part of an extracellular domain. Due to an action of the transmembrane region, the insulin receptor can be present as a membrane protein in a cell. A specific configuration of the β subunit in the present disclosure is not particularly limited, and any known β subunit can be adopted.

The first region according to the present disclosure does not include the transmembrane region (that is, may be a soluble protein (non-membrane protein)). Therefore, the insulin binding protein can be easily purified, and a protein with high purity can be obtained. The first region can easily bind to insulin in a sample containing a large amount of aqueous solution or water to form a complex (insulin-insulin binding protein complex).

In the present specification, the term "transmembrane region" refers to a partial region in a membrane protein that is embedded in a cell membrane when the membrane protein is disposed on the cell membrane. Many of transmembrane regions have a structure formed by a hydrophobic amino acid (for example, an α-helix structure formed by a hydrophobic amino acid), but are not limited to this structure.

The number of amino acids in the first region according to the present disclosure is, for example, 16, but the number of amino acids is not limited thereto. The first region may be a sequence containing an α-CT segment (16 amino acids) and having a conformation exposed on a surface of the insulin binding protein.

The first region according to the present disclosure may further include a configuration other than the α-CT segment, but preferably, the first region is substantially formed of the α-CT segment. Here, "the first region is substantially formed of the α-CT segment" may include a case in which the first region is a sequence having comparable insulin binding ability as compared with that of a native α-CT segment.

Another configuration in a case in which the first region includes a configuration other than the α-CT segment of the insulin receptor is not limited as long as the first region does not include the transmembrane region. For example, there is a linkage section for linking the α-CT segment to an electrode or a linker described in detail below. Here, the linkage section may be a peptidic linker or a non-peptidic linker (for example, a crosslinking agent).

### (Second Region Including L1 Domain of Insulin Receptor and not Including β Subunit of Insulin Receptor (Second Region))

The second region is a polypeptide including the L1 domain of the insulin receptor. Here, the L1 domain is a partial region of the α subunit, which is the extracellular domain of the insulin receptor, has insulin binding ability, and does not include a transmembrane region. A specific configuration of the L1 domain in the present disclosure is not particularly limited, and any known L1 domain can be adopted.

The second region according to the present disclosure does not include the β subunit of the insulin receptor. The β subunit is an intracellular domain of the insulin receptor, and includes a transmembrane region and a part of an extracellular domain. Due to an action of the transmembrane region, the insulin receptor can be present as the membrane protein in the cell. The specific configuration of the β subunit in the present disclosure is not particularly limited, and any known β subunit can be adopted.

The second region according to the present disclosure does not include the transmembrane region (that is, may be a soluble protein (non-membrane protein)). Therefore, the insulin binding protein can be easily purified, and a protein with high purity can be obtained. The second region can easily recognize insulin and the first region in a sample containing a large amount of aqueous solution or water to form a complex (insulin-insulin binding protein complex).

The second region according to the present disclosure may further include a configuration other than the L1 domain, but preferably, the second region is substantially formed of the L1 domain. Here, "the second region is substantially formed of the L1 domain" may include a sequence having comparable insulin binding ability as compared with that of a native L1 domain.

The second region according to the present disclosure may further include a CR domain of the insulin receptor. The CR domain is a cysteine rich region adjacent to the L1 domain. A specific configuration of the CR domain in the present disclosure is not particularly limited, and any known CR domain can be adopted. Preferably, the second region is substantially formed of the L1 domain and the CR domain. Here, "the second region is substantially formed of the L1 domain and the CR domain" may include a sequence having comparable insulin binding ability as compared with that of the native L1 domain.

The number of amino acids in the second region according to the present disclosure is, for example, 155 or more which is the same as the number of amino acids in the L1 domain. The number of amino acids in a case in which the second region also includes the CR domain described later is, for example, 310 or more.

Another configuration in a case in which the second region includes a configuration other than the L1 domain of the insulin receptor is not limited as long as the second region does not include the transmembrane region. For example, there is a linkage section for linking the L1 domain to the electrode or the linker described in detail below. Here, the linkage section may be a peptidic linker or a non-peptidic linker (for example, a crosslinking agent).

### (Arrangement of First Region and Second Region)

The insulin binding protein according to the present disclosure is immobilized on the surface of the electrode in one of the first region and the second region. Here, immobilizing of the insulin binding protein on the surface of the electrode is such that one end side of one of the first region and the second region is immobilized on the surface of the electrode and the other end is in a free state (non-immobilized state). Specific examples include (a) and (b), in which (a) is a form in which the first region (α-CT segment) and the second region (L1 domain) are connected to form the insulin binding protein (L1 domain-(if necessary, linker)-α-CT segment), and a first region (α-CT segment) side is immobilized on the surface of the electrode (L1 domain-(if necessary, linker)-α-CT segmentelectrode); and (b) is a form in which the first region (α-CT segment) and the second region (L1 domain) are connected to form the insulin binding protein (α-CT segment-(if necessary, linker)-L1 domain), and a second region (L1 domain) side is immobilized on the surface of the electrode (α-CT segment-(if necessary, linker)-L1 domain-electrode). In (a) and (b), (a) is preferable. Thus, the first region (α-CT segment) and the second region (L1 domain) are connected via the linker, and an end portion of one of the first region and the second region is immobilized on the surface of the electrode, so that each of the two regions bound via the linker binds so as to sandwich insulin, thereby forming an insulin-insulin binding protein complex. Therefore, the insulin can be captured more efficiently and more reliably, and a structural change associated with insulin binding can be captured more reliably as an electrical change. Therefore, a smaller amount (lower concentration) of insulin can be detected with higher accuracy. In particular, in the form (a), the L1 domain having a larger molecular weight than that of the α-CT segment is present on a non-immobilized end side of the insulin binding protein. Therefore, the insulin binding protein immobilized on the electrode has a large difference in a three-dimensional structure between an insulin-unbound state and an insulin-bound state. Therefore, it is possible to more sensitively capture a change in an electric signal associated with the formation of the complex of the insulin and the insulin binding protein, and it is possible to detect a further smaller amount (further lower concentration) of insulin.

As defined in (a) and (b) above, the first region (α-CT segment) and the second region (L1 domain) are preferably proteins connected to each other via the linker. According to the forms, the linker can appropriately adjust a distance between the first region (α-CT segment) and the second region (L1 domain). Therefore, the second region can more efficiently recognize the first region and insulin bound to an insulin binding site in the first region. Accordingly, the insulin can be more efficiently and more reliably bound in the first region (α-CT segment) and the second region (L1 domain). Therefore, according to the forms, the insulin can be detected with higher accuracy even for a smaller amount (lower concentration) of insulin. That is, in a preferred aspect of the present disclosure, the first region and the second region are connected via the linker. In a more preferred aspect of the present disclosure, the first region and the second region are connected via the linker, and the second region is immobilized on the surface of the electrode, or the first region and the second region are connected via the linker, and the first region is immobilized on the surface of the electrode. In a particularly preferred aspect of the present disclosure, the first region and the second region are connected via the linker, and the first region is immobilized on the surface of the electrode.

In the aspect in which the first region and the second region are connected via the linker, the linker that can be used is not particularly limited as long as the linker can bind the first region and the second region, and can be appropriately selected according to structures of the first region and the second region to be used. Specifically, preferred examples of the linker include a polypeptide. By using the polypeptide as the linker thus, it is possible not only to implement a structure capable of freely bending the first region and the second region so that the first region and the second region are disposed at appropriate positions with respect to the insulin, but also to construct an insulin binding protein as one protein (a fusion protein from the first region-the linker-the second region). Therefore, in this aspect, the fusion protein can be easily prepared by preparing an expression vector having a desired fusion protein by a known method and introducing the expression vector into a desired host.

When the polypeptide is used as the linker, the number of amino acids forming the linker is preferably 15 or more, and more preferably 20 or more, from the viewpoint of detecting insulin at a lower concentration. When the linker has such a length, the α-CT segment in the first region can more efficiently recognize the insulin. In addition, when the insulin-insulin binding protein complex is formed, the insulin can be sandwiched so that the first region (α-CT segment) and the second region (L1 domain) wrap around the insulin. Accordingly, an electrochemical change caused by a structural change of the insulin binding protein in the vicinity of the surface of the electrode can be grasped with higher sensitivity. Therefore, according to this aspect, the insulin can be detected with high sensitivity even for a smaller amount (lower concentration) of insulin. An upper limit of the number of amino acids forming the linker is not particularly limited, but is 50 or less, and preferably 40 or less, from the viewpoint of reactivity and the like.

When the linker is too short (for example, the number of amino acids forming the linker is less than 15), there is a possibility that the first region and the second region cannot recognize to sandwich the insulin. From the viewpoint that the insulin can be recognized more sensitively by securing a distance for disposing the insulin between the first region and the second region by the linker, the linker preferably has the length described above.

When the polypeptide is used as the linker, a type of an amino acid forming the linker is not particularly limited. From the viewpoint of measuring a smaller amount (lower concentration) of insulin, the linker is preferably formed of an amino acid having a high degree of freedom in a three-dimensional structure. More specifically, the linker preferably contains at least one amino acid selected from a group consisting of (i) an amino acid having a side chain of a fatty acid, such as glycine, alanine, valine, leucine, and isoleucine; and (ii) an amino acid having a side chain containing a hydroxyl group, such as serine, threonine, and tyrosine. In addition to the above, the linker may have (iii) an amino acid having a side chain containing an acid or amide, such as aspartic acid, asparagine, glutamic acid, and glutamine. Preferably, the linker is formed of at least one amino acid selected from a group consisting of the above (i) and at least one amino acid selected from a group consisting of the above (ii). More preferably, the linker is formed of at least one amino acid selected from the group consisting of (i), at least one amino acid selected from the group consisting of (ii), and at least one amino acid selected from a group consisting of (iii).

In the present disclosure, a method of preparing the first region (α-CT segment), the second region (L1 domain), or a complex of the first region (α-CT segment) and the second region (L1 domain) (α-CT segment-(if necessary, linker)-L1 domain complex) (hereinafter, also collectively referred to as "complex or the like") is not particularly limited, and a known method can be used in a similar manner, or a known method can be appropriately modified. For example, an expression vector containing a complex or the like may be introduced into a cell, a desired complex or the like may be expressed intracellularly, and the desired complex or the like may be secreted into a medium. In order to induce and promote the secretion, it is preferable to bind an extracellular secretion signal to a C-terminus or an N-terminus of the complex or the like. Alternatively, a complex or the like may be expressed as a tagged protein (for example, a histidine tag or a GST fusion protein) so as to facilitate purification.

### (Electrode)

An end portion having one of the first region and the second region according to the present disclosure is immobilized on the surface of the electrode. Here, the electrode is not particularly limited, and can be appropriately selected depending on an electrochemical detection method of the insulin-insulin binding protein complex. Specifically, a metal electrode such as a gold electrode or a platinum electrode, or a carbon electrode can be used.

A method of immobilizing the first region or the second region on the surface of the electrode is not particularly limited, and can be applied in a similar manner as a known method, or by appropriately modifying a known method. For example, in a case of binding to a noble metal electrode including a gold electrode, a known method can be used such as a method of adding a known gold binding peptide (for example, amino acid sequence: MHGKTQATSGTIQS) to an end of a polypeptide on a side to be immobilized on the electrode, and a method of adding a sulfur-containing amino acid residue having gold-binding ability (for example, a cysteine residue or a methionine residue) to the end of the polypeptide on the side to be immobilized on the electrode. A repetitive sequence of the gold binding peptide can be used for immobilization on the electrode with a desired binding force. Depending on this immobilizing method, a surface treatment of the electrode (for example, surface polishing, cyclic voltammetry treatment with an acid solution, or washing) may be performed.

A size and shape of the electrode are not particularly limited, and can be appropriately selected according to a use and the like.

### (Electrochemical Detection)

In the present disclosure, a sample is added to the electrode (insulin binding protein-immobilized electrode) prepared as described above in which the binding protein specifically recognizing a site of the insulin is immobilized on the surface, and an electrochemical change associated with formation of the insulin-insulin binding protein complex is detected. Here, the electrochemical detection method is not particularly limited, and a known method can be used in a similar manner, or a known method can be appropriately modified. For example, the electrochemical impedance spectroscopy (EIS) or the like can be used. In the electrochemical detection method, the electrochemical impedance spectroscopy (EIS) can be preferably used. That is, in a preferred aspect of the present disclosure, the electrochemical change associated with the formation of the complex of the insulin and the insulin binding protein is detected by the electrochemical impedance spectroscopy (EIS) (the insulin-insulin binding protein complex is electrochemically detected by the electrochemical impedance spectroscopy (EIS)). In the electrochemical impedance spectroscopy (EIS), AC voltage pertubation with a small sine wave amplitude (for example, about 10 mV) can be applied between two electrodes, a current obtained between the electrodes can be measured as a function of an AC frequency, and an impedance can be calculated as a function of a frequency. Such a change in an impedance spectrum is preferable because the change can be measured with high sensitivity without using a label.

Here, signal intensity is electrochemically detected using a known amount (concentration) of insulin (for example, impedance is measured in EIS), and a calibration curve is created. By comparing signal intensities (for example, the impedance in EIS) detected in the sample based on the calibration curve, an amount of insulin (insulin concentration) in the sample can be accurately measured. That is, according to a preferred aspect of the present disclosure, using insulin whose amount (concentration) is understood in advance, a calibration curve for the signal intensity with respect to the amount (concentration) of insulin is created, and based on the calibration curve and a detected signal intensity in the sample, the amount (concentration) of insulin contained in the sample is measured.

For example, by creating the calibration curve using signal intensity (for example, relative impedance) obtained using a standard sample containing insulin at an understood concentration, the amount (concentration) of insulin contained in a sample can be simply quantified. The calibration curve obtained by the present disclosure is effective as a calibration curve because the calibration curve is a constant calibration curve regardless of external factors. Therefore, by using this calibration curve, the amount of insulin actually present in the sample can be accurately measured. The detection method above is a fairly useful assay method in clinical examination.

### <Insulin Detection Kit (Second Aspect of Present Disclosure) >

According to the second aspect of the present disclosure, there is provided an insulin detection kit including an electrode having an insulin binding protein immobilized on a surface of the electrode, the insulin binding protein specifically recognizing a site of insulin. The insulin binding protein includes a first region that includes an α-CT segment of an insulin receptor and does not include a β subunit of the insulin receptor and a second region that includes an L1 domain of the insulin receptor and does not include the β subunit of the insulin receptor, and one of the first region and the second region is immobilized on the surface of the electrode. In the present aspect, the "insulin binding protein", the "α-CT" and the "L1 domain" and (if necessary) a "CR domain" that constitute the insulin binding protein, the first region, the second region, and the electrode are defined in a similar manner as in the first aspect, and thus the description thereof is omitted here.

The electrode on the surface of which the insulin binding protein including the first region (α-CT segment) and the second region (L1 domain) is immobilized may be placed in one container. Alternatively, the electrode may be placed in the container together with a buffer solution. The detection kit may contain the electrode solution and a buffer solution for an assay or a concentrated stock solution thereof.

The detection kit according to the present aspect may further include a standard substance whose amount (concentration) of insulin is understood and a container containing the standard substance. Each reagent included in the detection kit may be provided by being dispensed into a container for each sample measurement, or a plurality of sample measurements may be provided in individual containers, collectively provided for each reagent. In the latter case, each reagent is dispensed into a predetermined container for measurement at the time of use. When the reagent is provided for each sample measurement, the container containing the reagent may be integrally molded as a cartridge, or the reagent may be stored in a different section of the cartridge. The container that may be included in the detection kit may be made of any material as long as the material does not interact with the first region or the second region and does not interfere with reaction used for detection, for example, insulin-insulin binding protein complex formation reaction. If necessary, a surface of the container may be pre-treated and provided so as not to cause such an interaction. A manual of handling is usually attached to the detection kit.

### [Examples]

Effects of the invention will be described with reference to the following Examples and Comparative Examples. However, a technical scope of the invention is not limited to the following Examples. In the following Examples, unless otherwise specified, an operation was performed at room temperature (25°C). Unless otherwise specified, "%" and "parts" mean "% by weight" and "parts by weight", respectively.

### <Synthesis Example 1: Preparation of Protein of Example 1>

An amino acid sequence in Example 1 (hereinafter, for simplification, a protein of Example 1 is also referred to as "3GαL" or "3GαL protein") is shown in a following amino acid sequence (SEQ ID NO: 1). In the following amino acid sequences, a single underlined portion (amino acid sequence: METDTLLLWVLLLWVPGSTGDQL) represents an extracellular secretion signal; a point underlined portion (amino acid sequence: MHGKTQATSGTIQS triple-repetition structure) represents a gold binding peptide (GBP); a hollow portion (amino acid sequence: TFEDYLHNVVFVPRPS) represents an insulin receptor-α-CT segment; a long point underlined portion (amino acid sequence: ASSAGGSAGGSAGGSAGGSAGGSAGGSAGSGGLE) represents a linker; a double underlined portion (amino acid sequence: HLYPGEVCPGMDIRNNLTRLHELENCSVIEGHLQILLMFKTRPEDFRDLSFPKLIMI TDYLLLFRVYGLESLKDLFPNLTVIRGSRLFFNYALVIFEMVHLKELGLYNLMNITR GSVRIEKNNELCYLATIDWSRILDSVEDNYIVLNKDDNEECGDVCPGTAKGKTNCPA TVINGQFVERCWTHSHCQKVCPTICKSHGCTAEGLCCHKECLGNCSEPDDPTKCVAC RNFYLDGQCVETCPPPYYHFQDWRCVNFSFCQDLHFKCRNSRKPGCHQYVIHNNKCI PECPSGYTMNSSNLMCTPCLGPCPK) represents an insulin receptor-L1 domain and CR domain; and an embossed portion (amino acid sequence: HHHHHH) represents a histidine tag. In the following amino acid sequence, the histidine tag is linked to a C-terminus of the L1 domain-the CR domain in order to facilitate purification. Since the extracellular secretion signal is cleaved between METDTLLLWVLLLWVPGSTG and D when secreted, the protein (3GαL) obtained after purification is considered to have an amino acid sequence starting from DQLM in SEQ ID NO: 1 below.

### [Chem 1]

SEQ ID 1:

METDTLLLWVLLLWVPGSTGDQLMHGKTQATSGTIQSMHGKTQATSGTIQS
MHGKTQATSGTIQSMTFEDYLHNVVFVPRPSASSAGGSAGGSAGGSAGGSA
GGSAGGSAGSGGLEHLYPGEVCPGMDIRNNLTRLHELENCSVIEGHLQILL
MFKTRPEDFRDLSFPKLIMITDYLLLFRVYGLESLKDLFPNLTVIRGSRLFF
NYALVIFEMVHLKELGLYNLMNITRGSVRIEKNNELCYLATIDWSRILDSVE
DNYIVLNKDDNEECGDVCPGTAKGKTNCPATVINGQFVERCWTHSHCQKV
CPTICKSHGCTAEGLCCHKECLGNCSEPDDPTKCVACRNFYLDGQCVETCP
PPYYHFQDWRCVNFSFCQDLHFKCRNSRKPGCHQYVIHNNKCIPECPSGY
TMNSSNLMCTPCLGPCPKSRTGHHHHHH

First, according to a known method, a DNA sequence (SEQ ID NO: 2) corresponding to the amino acid sequence of SEQ ID NO: 1 was amplified, and the DNA sequence was cloned into an expression plasmid for animal cells (pcDNA3.1/myc-His B (Life Technologies)). In a histidine tag portion, a DNA sequence derived from an expression plasmid was used.

### [Chem 2]

SEQ ID 2:

ATGGAGACAGACACACTCCTGCTATGGGTACTGCTGCTCTGGGTTCCAGGT
TCCACTGGTGACCAGCTTATGCATGGGAAAACACAAGCCACAAGTGGCAC
CATTCAGTCTATGCATGGTAAGACCCAGGCAACGTCAGGCACAATCCAGTC
AATGCACGGTAAAACTCAGGCCACTTCCGGCACAATACAGAGTATGACGTT
TGAGGATTACCTCCACAATGTCGTGTTTGTGCCCAGACCAAGTGCTAGCTC
CGCTGGTGGATCAGCTGGAGGTTCCGCTGGAGGAAGCGCCGGTGGTTCTG
CCGGAGGGAGTGCAGGCGGGAGCGCAGGGAGCGGAGGGCTGGAACATCT
GTACCCAGGAGAAGTCTGCCCTGGCATGGACATCCGGAATAACCTGACCA
GGCTTCACGAGCTGGAGAATTGTAGCGTGATCGAAGGGCACTTGCAGATC
TTGTTGATGTTTAAAACTCGCCCTGAGGATTTCCGGGATCTGTCTTTTCCTA
AGCTGATCATGATTACTGACTACCTGTTGCTGTTCCGCGTGTATGGGCTCG
AGTCCCTGAAGGACCTCTTTCCCAATCTGACCGTGATTAGAGGGAGTCGGC
TCTTCTTCAACTACGCGCTGGTGATTTTCGAGATGGTCCACCTGAAGGAGC
TTGGGCTGTATAACCTGATGAACATAACCCGAGGGTCTGTCAGGATCGAGA
AGAACAACGAGCTGTGCTATCTGGCCACAATTGACTGGTCCCGTATCCTCG
ATAGCGTTGAGGACAATTACATTGTCCTTAACAAGGACGACAACGAAGAAT
GTGGCGATGTATGTCCCGGCACTGCCAAAGGCAAAACCAACTGCCCTGCC
ACAGTTATTAATGGCCAGTTTGTGGAAAGATGTTGGACCCACTCACACTGT
CAGAAGGTTTGCCCCACTATCTGCAAATCTCATGGATGCACTGCAGAAGGC
CTCTGTTGCCATAAGGAGTGTCTGGGCAATTGTTCAGAGCCTGATGATCCC
ACAAAGTGCGTAGCGTGCCGAAATTTCTACCTGGACGGACAGTGTGTTGAA
ACCTGTCCGCCACCCTACTATCATTTCCAGGATTGGCGCTGTGTGAATTTC
AGCTTTTGCCAAGACCTCCACTTCAAGTGCAGGAACTCCAGGAAACCAGG
CTGCCATCAATATGTGATCCACAACAACAAATGCATACCGGAATGCCCAAG
CGGCTATACGATGAATTCCAGCAATCTGATGTGCACCCCATGTCTTGGACC
TTGTCCCAAGTCTAGAACCGGT

Next, the thus obtained plasmid and Expi293 Expression System (manufactured by Thermo Fisher Scientific) were used to secrete and express the 3GαL protein in a medium. The 3GαL protein is released into the medium by a secretion signal, and an extracellular secretion signal portion is cleaved.

Specifically, first, Expi293 cells (7.5 × 10⁷ cells) in culture using 30 mL of medium were transfected using the plasmid obtained above according to a protocol of the manufacturer. After culture for 7 days after transfection, about 30 mL of the medium was collected, centrifuged under the conditions of 4,000 × g for 10 minutes at 4°C, and supernatant of a culture solution was collected. Next, the supernatant was dialyzed in a dialysis buffer (PBS, pH 7.4). At this time, after 1 hour and 2 hours of dialysis, the dialysis buffer (PBS, pH 7.4) was exchanged, and dialysis was further performed for 17 hours.

Next, the supernatant was subjected to a HisTrap FF crude column (manufactured by GE Healthcare Living Science (Cytiva)), and a target protein (3GαL protein) contained in the dialyzed supernatant was adsorbed on the HisTrap FF column (HisTrap FF crude column). Thereafter, the target protein (3GαL protein) was eluted from the HisTrap FF column by an imidazole concentration gradient method. After elution and purification, an eluted substance was dialyzed again in a dialysis buffer (PBS, pH 7.4). At this time, after 1 hour and 2 hours of dialysis, dialysis buffer (PBS, pH 7.4) was exchanged, and dialysis was further performed for 18 hours, and a solution containing 3GαL protein was obtained. After dialysis, a protein concentration was determined using Micro BCA^{™}Protein Assay Kit (manufactured by Thermo Fisher Scientific) and stored at 4°C.

### <Synthesis Example 2: Preparation of Protein of Example 2>

An amino acid sequence in Example 2 (hereinafter, for simplification, a protein of Example 2 is also referred to as "αL3G" or "αL3G protein") is shown in a following amino acid sequence (SEQ ID NO: 3).

In the following amino acid sequences, a single underlined portion (amino acid sequence: METDTLLLWVLLLWVPGSTGDQL) represents an extracellular secretion signal; a hollow portion (amino acid sequence: MTFEDYLHNVVFVPRPS) represents an insulin receptor-α-CT segment; a long point underlined portion (amino acid sequence: ASSAGGSAGGSAGGSAGGSAGGSAGGSAGSGGLE) represents a linker; a double underlined portion (amino acid sequence: HLYPGEVCPGMDIRNNLTRLHELENCSVIEGHLQILLMFKTRPEDFRDLSFPKLIMI TDYLLLFRVYGLESLKDLFPNLTVIRGSRLFFNYALVIFEMVHLKELGLYNLMNITR GSVRIEKNNELCYLATIDWSRILDSVEDNYIVLNKDDNEECGDVCPGTAKGKTNCPA TVINGQFVERCWTHSHCQKVCPTICKSHGCTAEGLCCHKECLGNCSEPDDPTKCVAC RNFYLDGQCVETCPPPYYHFQDWRCVNFSFCQDLHFKCRNSRKPGCHQYVIHNNKCI PECPSGYTMNSSNLMCTPCLGPCPK) represents an insulin receptor-L1 domain and CR domain; a point underlined portion (amino acid sequence: MHGKTQATSGTIQS triple-repetition structure) represents a gold binding peptide (GBP); and an embossed portion (amino acid sequence: HHHHHH) represents a histidine tag. In the following amino acid sequence, the histidine tag is linked to a C-terminus of the GBP in order to facilitate purification. Since the extracellular secretion signal is cleaved between METDTLLLWVLLLWVPGSTG and D when secreted, the protein (αL3G) obtained after purification is considered to have an amino acid sequence starting from DQLM in SEQ ID NO: 3 below.

### [Chem 3]

SEQ ID 3:

METDTLLLWVLLLWVPGSTGDQLMTFEDYLHNVVFVPRPSASSAGGSAGG
SAGGSAGGSAGGSAGGSAGSGGLEHLYPGEVCPGMDIRNNLTRLHELENCS
VIEGHLQILLMFKTRPEDFRDLSFPKLIMITDYLLLFRVYGLESLKDLFPNLT
VIRGSRLFFNYALVIFEMVHLKELGLYNLMNITRGSVRIEKNNELCYLATID
WSRILDSVEDNYIVLNKDDNEECGDVCPGTAKGKTNCPATVINGQFVERCW
THSHCQKVCPTICKSHGCTAEGLCCHKECLGNCSEPDDPTKCVACRNFYLD
GQCVETCPPPYYHFQDWRCVNFSFCQDLHFKCRNSRKPGCHQYVIHNNKC
IPECPSGYTMNSSNLMCTPCLGPCPKSRMHGKTQATSGTIQSMHGKTQATS
GTIQSMHGKTQATSGTIQSTGHHHHHH

First, according to a known method, a DNA sequence (SEQ ID NO: 4) corresponding to the amino acid sequence of SEQ ID NO: 3 was amplified, and the DNA sequence was cloned into an expression plasmid for animal cells (pcDNA3.1/myc-His B (Life Technologies)). In a histidine tag portion, a DNA sequence derived from an expression plasmid was used.

### [Chem 4]

SEQ ID 4:

ATGGAGACAGACACACTCCTGCTATGGGTACTGCTGCTCTGGGTTCCAGGT
TCCACTGGTGACCAGCTTATGACCTTTGAGGACTATCTGCACAATGTCGTT
TTCGTGCCACGACCCTCAGCCTCCAGTGCCGGTGGATCCGCTGGAGGGTC
CGCAGGCGGAAGCGCCGGCGGGTCAGCTGGTGGAAGTGCTGGTGGGTCT
GCTGGCAGCGGCGGGCTGGAGCATCTTTATCCAGGCGAAGTGTGTCCCGG
AATGGACATCCGTAACAATCTGACCAGACTGCACGAACTCGAGAACTGCTC
TGTCATCGAAGGGCATCTCCAGATTCTGCTGATGTTCAAAACGAGGCCCGA
AGATTTCCGGGACCTGTCATTCCCCAAACTGATCATGATTACTGATTATCTG
CTCCTCTTTCGCGTTTACGGGCTGGAATCCCTTAAAGACCTCTTTCCAAAT
CTGACAGTGATTCGGGGATCTCGCTTGTTCTTCAACTATGCCCTTGTCATC
TTCGAGATGGTGCACTTGAAGGAACTGGGACTCTACAACTTGATGAACATA
ACTAGAGGTTCCGTAAGAATAGAGAAGAATAATGAGCTGTGCTACTTGGCA
ACCATCGATTGGTCAAGGATACTGGATAGCGTCGAGGACAACTACATCGTG
CTGAACAAAGACGATAACGAGGAGTGTGGGGACGTTTGCCCAGGCACTGC
AAAGGGCAAGACCAATTGCCCTGCCACAGTGATTAATGGCCAGTTTGTGGA
GAGATGCTGGACTCACTCCCATTGCCAGAAAGTCTGTCCAACGATCTGTAA
AAGTCATGGCTGCACAGCCGAGGGACTGTGCTGCCATAAGGAATGCCTCG
GGAATTGCAGTGAACCTGACGATCCCACCAAGTGTGTTGCCTGTCGCAACT
TTTACCTGGATGGGCAATGTGTAGAAACTTGTCCGCCACCCTATTACCATTT
CCAGGATTGGAGGTGTGTGAACTTCAGCTTTTGCCAAGACCTTCACTTTAA
ATGTAGGAACTCCCGGAAGCCTGGCTGCCACCAGTACGTGATTCACAATAA
CAAGTGCATTCCTGAGTGTCCGTCAGGGTATACCATGAATAGCAGCAATCT
GATGTGTACACCCTGTCTGGGCCCTTGCCCTAAGAGCCGAATGCACGGAAA
GACTCAGGCAACAAGTGGCACAATCCAGAGCATGCATGGTAAGACCCAAG
CGACCTCTGGTACCATCCAGTCTATGCACGGCAAAACACAGGCGACTAGCG
GAACGATTCAGTCTACCGGT

Next, the thus obtained plasmid and Expi293 Expression System (manufactured by Thermo Fisher Scientific) were used to secrete and express the αL3G protein in a medium. The αL3G protein is released into the medium by a secretion signal, and an extracellular secretion signal portion is cleaved.

Specifically, first, Expi293 cells (7.5 × 10⁷ cells) in culture using 30 mL of medium were transfected using the plasmid obtained above according to a protocol of the manufacturer. After culture for 7 days after transfection, about 30 mL of the medium was collected, centrifuged under the conditions of 4,000 × g for 10 minutes at 4°C, and supernatant of a culture solution was collected. Next, the supernatant was dialyzed in a dialysis buffer (PBS, pH 7.4). At this time, after 1 hour and 2 hours of dialysis, the dialysis buffer (PBS, pH 7.4) was exchanged, and dialysis was further performed for 17 hours.

Next, the supernatant was subjected to a HisTrap FF crude column (manufactured by GE Healthcare Living Science (Cytiva)), and a target protein (αL3G protein) contained in the dialyzed supernatant was adsorbed on the HisTrap FF column (HisTrap FF crude column). Thereafter, the target protein (αL3G protein) was eluted from the HisTrap FF column by an imidazole concentration gradient method. After elution and purification, an eluted substance was dialyzed again in a dialysis buffer (PBS, pH 7.4). At this time, after 1 hour and 2 hours of dialysis, dialysis buffer (PBS, pH 7.4) was exchanged, and dialysis was further performed for 18 hours, and a solution containing αL3G protein was obtained. After dialysis, a protein concentration was determined using Micro BCA^{™}Protein Assay Kit (manufactured by Thermo Fisher Scientific) and stored at 4°C.

### <Example 1: Preparation of 3GαL Electrode >

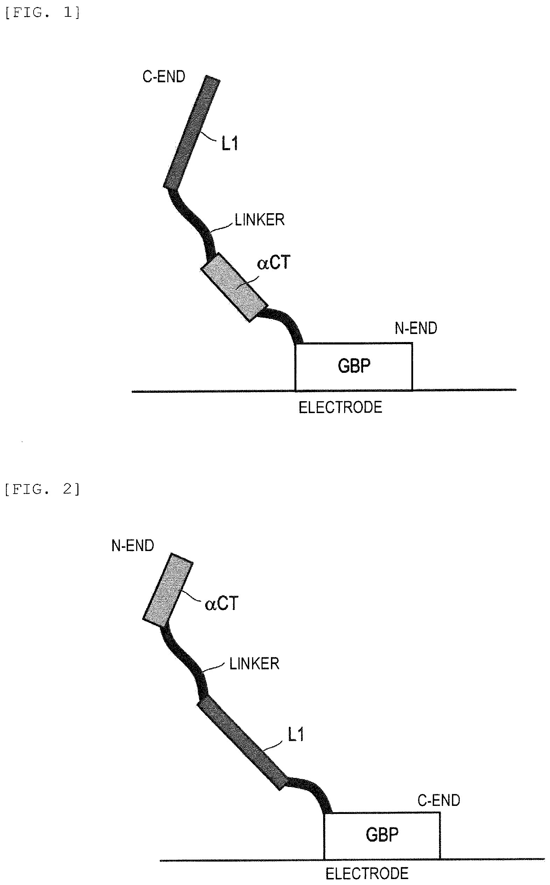

An electrode (3GαL electrode) having the 3GαL protein immobilized on the surface of the electrode as shown in Fig. 1 was prepared by a following method.

A solution containing the 3GαL protein obtained in Synthesis Example 1 was diluted with PBS (pH 7.4) to a concentration of 10 ng/µL, and a 3GαL solution was prepared.

A surface of an electrode of a SAUE gold electrode (manufactured by BAS, outer diameter: 3 mm, inner diameter: 1.6 mm) was polished using 0.05 µm polishing alumina (manufactured by BAS). Then, cyclic voltammetry was performed in a 1 N sulfuric acid aqueous solution until an oxidation-reduction wave in a clean state was obtained (potential range: 0 V to 1.6 V vs. Ag/AgCl, scan rate: 100 mV/s). Thereafter, the electrode was washed three times with 1 mL of MilliQ to prepare a gold electrode.

On a surface of the gold electrode immediately after washing, 4 µL of the 3GαL solution of 10 ng/µL prepared above was dropped, and the 3GαL protein was accumulated on the gold electrode at 4°C for 2 hours, and the 3GαL electrode was obtained.

### <Example 2: Preparation of αL3G Electrode>

An electrode (αL3G electrode) having an αL3G immobilized on the surface of the electrode as shown in Fig. 2 was prepared by a following method.

A solution containing the αL3G protein obtained in Synthesis Example 2 was diluted with PBS (pH 7.4) to a concentration of 10 ng/µL, and an αL3G solution was prepared.

A surface of an electrode of a SAUE gold electrode (manufactured by BAS, outer diameter: 3 mm, inner diameter: 1.6 mm) was polished using 0.05 µm polishing alumina (manufactured by BAS). Then, cyclic voltammetry was performed in a 1 N sulfuric acid aqueous solution until an oxidation-reduction wave in a clean state was obtained (potential range: 0 V to 1.6 V vs. Ag/AgCl, scan rate: 100 mV/s). Thereafter, the electrode was washed three times with 1 mL of MilliQ to prepare a gold electrode.

On a surface of the gold electrode immediately after washing, 4 µL of the αL3G solution of 10 ng/pl prepared above was dropped, and the αL3G protein was accumulated on the gold electrode at 25°C for 17 hours, and the αL3G electrode was obtained.

### <Electrode Evaluation >

With respect to the 3GαL electrode and the αL3G electrode obtained in Examples 1 and 2, insulin was detected by a following method.

The 3GαL electrode and the αL3G electrode were washed three times with 1 mL of PBS (pH 7.4). Next, each electrode was immersed in a PBS solution (pH 7.4) containing 5 mM of [Fe (CN)₆]^{3-/4-} for 1 hour. After immersion for a predetermined time, insulin was detected at each electrode by electrochemical impedance spectroscopy (EIS) measurement using an electrochemical measurement system HZ7000 (manufactured by Hokuto Denko Corporation). The EIS measurement above was performed under the conditions of measurement frequency = 100 mHz to 100 kHz and AC amplitude = Rest potential ± 10 mV. Insulin concentration dependency was evaluated as follows. First, the impedance was measured in PBS containing 5 mM of [Fe(CN) ₆]^{3-/4-}. At this time, the impedance at 10 Hz was used as a reference. Next, insulin was sequentially added to PBS containing 5 mM of [Fe(CN)₆]^{3-/4-} so that the final insulin concentration in the solution was 1 nM, 10 nM, 100 nM, and 1000 nM, and an insulin aqueous solution was prepared. Each electrode was immersed in each insulin aqueous solution for 10 minutes, and then the EIS measurement was performed in a similar manner as described above. The impedance at 10 Hz was evaluated as a relative value (relative impedance).

Results are shown in Fig. 3 (3GαL electrode) and Fig. 4 (αL3G electrode).

As shown in Figs. 3 and 4, it can be seen that the relative impedance of both the 3GαL electrode (Fig. 3) and the αL3G electrode (Fig. 4) increases as the insulin concentration increases. Accordingly, it can be seen that insulin can be detected by using the electrode according to the invention even when the insulin concentration in the sample is a trace amount (for example, 10 nM or less). It is considered that the insulin concentration in the sample can be quantified by using the electrode according to the invention. A comparison between Fig. 3 and Fig. 4 shows that the 3GαL electrode exhibits a higher relative impedance at a lower concentration. Therefore, the 3GαL electrode is suitable for detecting insulin at a lower concentration than the αL3G electrode, and has a sufficient possibility of detecting insulin contained in blood, for example.

The application is based on Japanese Patent Application No. 2020-094490, filed on May 29, 2020, the disclosure of which is referenced and incorporated as a whole.

## Claims

1. An insulin detection method comprising:
adding a sample to an electrode having an insulin binding protein immobilized on a surface of the electrode, the insulin binding protein specifically recognizing insulin; and
detecting an electrochemical change associated with formation of a complex of the insulin and the insulin binding protein, wherein
the insulin binding protein includes a first region that includes an α-CT segment of an insulin receptor and does not include a β subunit of the insulin receptor and a second region that includes an L1 domain of the insulin receptor and does not include the β subunit of the insulin receptor, and one of the first region and the second region is immobilized on the surface of the electrode.

2. The insulin detection method according to claim 1, wherein
the first region and the second region are connected via a linker, and the second region is immobilized on the surface of the electrode.

3. The insulin detection method according to claim 1, wherein
the first region and the second region are connected via a linker, and the first region is immobilized on the surface of the electrode.

4. The insulin detection method according to any one of claims 1 to 3, wherein
the second region further includes a CR domain of the insulin receptor.

5. The insulin detection method according to any one of claims 1 to 4, wherein
the electrochemical change associated with the formation of the complex of the insulin and the insulin binding protein is detected by electrochemical impedance spectroscopy (EIS).

6. An insulin detection kit comprising:
an electrode having an insulin binding protein immobilized on a surface of the electrode, the insulin binding protein specifically recognizing a site of insulin, wherein
the insulin binding protein includes a first region that includes an α-CT segment of an insulin receptor and does not include a β subunit of the insulin receptor and a second region that includes an L1 domain of the insulin receptor and does not include the β subunit of the insulin receptor, and one of the first region and the second region is immobilized on the surface of the electrode.

## Patentansprüche

1. Insulinnachweisverfahren mit den folgenden Schritten:
Zugabe einer Probe zu einer Elektrode mit einem auf einer Oberfläche der Elektrode immobilisierten Insulinbindungsprotein, wobei das Insulinbindungsprotein speziell Insulin erkennt, und
Erfassen einer elektrochemischen Veränderung in Verbindung mit der Bildung eines Komplexes aus dem Insulin und dem Insulinbindungsprotein, wobei
das Insulinbindungsprotein einen ersten Bereich aufweist, der ein α-CT-Segment eines Insulinrezeptors enthält und keine β-Untereinheit des Insulinrezeptors enthält, und einen zweiten Bereich, der eine L1-Domäne des Insulinrezeptors enthält und keine β-Untereinheit des Insulinrezeptors enthält, und wobei einer von dem ersten Bereich und dem zweiten Bereich auf der Oberfläche der Elektrode immobilisiert ist.

2. Insulinnachweisverfahren nach Anspruch 1, wobei
der erste Bereich und der zweite Bereich über einen Linker verbunden sind und der zweite Bereich auf der Oberfläche der Elektrode immobilisiert ist.

3. Insulinnachweisverfahren nach Anspruch 1, wobei
der erste Bereich und der zweite Bereich über einen Linker verbunden sind und der erste Bereich auf der Oberfläche der Elektrode immobilisiert ist.

4. Insulinnachweisverfahren nach einem der Ansprüche 1 bis 3, wobei
der zweite Bereich ferner eine CR-Domäne des Insulinrezeptors enthält.

5. Insulinnachweisverfahren nach einem der Ansprüche 1 bis 4, wobei
die elektrochemische Veränderung in Verbindung mit der Bildung des Komplexes aus dem Insulin und dem Insulinbindungsprotein durch elektrochemische Impedanzspektroskopie (EIS) nachgewiesen wird.

6. Insulinnachweiskit, das Folgendes umfasst:
eine Elektrode mit einem auf einer Oberfläche der Elektrode immobilisierten Insulinbindungsprotein, wobei das Insulinbindungsprotein speziell eine Insulinstelle erkennt, wobei
das Insulinbindungsprotein einen ersten Bereich enthält, der ein α-CT-Segment eines Insulinrezeptors enthält und keine β-Untereinheit des Insulinrezeptors enthält, und einen zweiten Bereich, der eine L1-Domäne des Insulinrezeptors enthält und keine β-Untereinheit des Insulinrezeptors enthält, und wobei einer von dem ersten Bereich und dem zweiten Bereich auf der Oberfläche der Elektrode immobilisiert ist.

## Revendications

1. Procédé de détection d'insuline comprenant :
l'ajout d'un échantillon à une électrode ayant une protéine de liaison à l'insuline immobilisée sur une surface de l'électrode, la protéine de liaison à l'insuline reconnaissant spécifiquement l'insuline ; et
la détection d'un changement électrochimique associé à la formation d'un complexe de l'insuline et de la protéine de liaison à l'insuline, dans lequel
la protéine de liaison à l'insuline inclut une première région qui inclut un segment α-CT d'un récepteur d'insuline et n'inclut pas une sous-unité β du récepteur d'insuline et une deuxième région qui inclut un domaine L1 du récepteur d'insuline et n'inclut pas la sous-unité β du récepteur d'insuline, et l'une de la première région et de la deuxième région est immobilisée sur la surface de l'électrode.

2. Procédé de détection d'insuline selon la revendication 1, dans lequel
la première région et la deuxième région sont reliées par un liant, et la deuxième région est immobilisée sur la surface de l'électrode.

3. Procédé de détection d'insuline selon la revendication 1, dans lequel
la première région et la deuxième région sont reliées par un liant, et la première région est immobilisée sur la surface de l'électrode.

4. Procédé de détection d'insuline selon l'une quelconque des revendications 1 à 3, dans lequel
la deuxième région inclut en outre un domaine CR du récepteur d'insuline.

5. Procédé de détection d'insuline selon l'une quelconque des revendications 1 à 4, dans lequel
le changement électrochimique associé à la formation du complexe de l'insuline et de la protéine de liaison à l'insuline est détecté par spectroscopie d'impédance électrochimique (EIS).

6. Kit de détection d'insuline comprenant :
une électrode ayant une protéine de liaison à l'insuline immobilisée sur une surface de l'électrode, la protéine de liaison à l'insuline reconnaissant spécifiquement un site d'insuline, dans lequel
la protéine de liaison à l'insuline inclut une première région qui inclut un segment α-CT d'un récepteur d'insuline et n'inclut pas une sous-unité β du récepteur d'insuline et une deuxième région qui inclut un domaine L1 du récepteur d'insuline et n'inclut pas la sous-unité β du récepteur d'insuline, et l'une de la première région et de la deuxième région est immobilisée sur la surface de l'électrode.
